# EUROPÄISCHE PATENTSCHRIFT

(11) **EP 3 192 432 B1**
(45) Veröffentlichungstag und Bekanntmachung des Hinweises auf die Patenterteilung: **15.11.2023**
(21) Anmeldenummer: 17151110.8
(22) Anmeldetag: 12.01.2017
(51) Int. Cl.: A61B 3/032, A61B 3/103, G02C 13/00

(54) **VERFAHREN ZUM ÜBERPRÜFEN DER AUGEN**
METHOD FOR TESTING EYES
PROCÉDÉ D'EXAMEN DES YEUX

(30) Priorität: 14.01.2016 DE 102016000233
(43) Veröffentlichungstag der Anmeldung: 19.07.2017
(73) Patentinhaber: Oculus Optikgeräte GmbH, 35582 Wetzlar (DE)
(72) Erfinder: STEINMÜLLER, Andreas, 35435 Wettenberg (DE); DEGLE, Prof. Dr. Stephan, 07743 Jena (DE)
(74) Vertreter: advotec.

(56) Entgegenhaltungen:
- EP-A1- 2 772 795
- WO-A1-2004/068216
- DE-A1-102011 009 646
- US-A1- 2015 374 224

## Beschreibung

Die Erfindung betrifft ein Verfahren zum Überprüfen der Augen eines Probanden mit einem Sehprüfsystem, wobei zumindest einem Auge des Probanden mittels eines Bildschirms eines Anzeigegeräts des Sehprüfsystems Sehprüfzeichen visualisiert werden, wobei mit einer Kamera einer Kameravorrichtung des Anzeigegeräts Augen des Probanden zusammen mit einer Brillenfassung des Sehprüfsystems aufgenommen werden, wobei mit einer Steuervorrichtung des Sehprüfsystems das Anzeigegerät gesteuert wird, wobei mit der Steuervorrichtung eine Position der Brillenfassung relativ zu den Augen mittels Bildverarbeitung bestimmt wird.

Derartige Sehprüfsysteme sind hinreichend bekannt und werden regelmäßig zur Durchführung von Sehtests genutzt. So verfügen die bekannten Anzeigegeräte beziehungsweise Bildschirme über ein Steuergerät, über welches eine Bedienperson eine Wiedergabe von Sehprüfzeichen auf dem Bildschirm steuern kann. Je nach Art des Bildschirms kann dieser auch über eine lineare oder zirkulare Polarisation verfügen. Die Polarisation des Bildschirms wird regelmäßig zur Durchführung von Sehtests in Verbindung mit einer Messbrille oder einem Phoropter genutzt. Weiter kann das Sehprüfsystem eine Kameravorrichtung mit einer Kamera umfassen, welche beispielsweise zur Vermessung der Augen des Probanden genutzt werden kann. Eine Bestimmung einer subjektiven Refraktion der Augen des Probanden erfolgt durch eine Darbietung verschiedenster Sehprüfzeichen durch eine Bedienperson des Sehprüfsystems in Verbindung mit Probegläsern eines Phoropters oder einer Messbrille. Dabei können auch verschiedene, allgemein bekannte Sehtests, unter anderem eine Testung eines Nahsehens und eines Fernsehens, mit dem Sehprüfsystem durchgeführt werden.

Wird bei einem derartigen Sehtest festgestellt, dass eine Fern- und Nahkorrektur von Fehlsichtigkeiten erforderlich ist, wird eine Korrektur dieser Fehlsichtigkeiten regelmäßig mittels sogenannter Gleitsichtgläser durchgeführt. Dabei ist es wesentlich, dass die Gleitsichtgläser in ihrer Position relativ zum Auge entsprechend einer angenommenen Sehaufgabe mit einer Brillenfassung beziehungsweise einem Brillengestell angeordnet sind. Beispielsweise ist bei einem Fernsehen eine Sehachse des Auges im Wesentlichen horizontal und bei einem Nahsehen relativ zu einer Horizontalen durch eine Augenbewegung geneigt ausgerichtet. Ein Gleitsichtglas beziehungsweise Brillenglas zur Fern- und Nahkorrektur muss nun so relativ zum Auge ausgerichtet sein, dass ein Fernbereich des Gleitsichtglases im Bereich einer horizontal ausgerichteten Sehachse beziehungsweise ein Nahbereich des Gleitsichtglases im Bereich einer geneigt ausgerichteten Sehachse des Auges liegt. Diese Positionierung beziehungsweise Anpassung des Gleitsichtglases erfolgt regelmäßig dadurch, dass manuell eine Relativposition der Pupille zu einem an einen Kopf des Probanden angepassten Brillengestell vermessen wird. So wird es möglich, eine Positionsmarke an dem Gleitsichtglas anzubringen, die mit einer Sehachse übereinstimmt und von der ausgehend dann eine Außenkontur des Gleitsichtglases, die der Brillenfassung angepasst ist, an dem Gleitsichtglas geschliffen werden kann.

Bei dem bekannten Verfahren ist es insbesondere nachteilig, dass nach der Durchführung des Sehtests, insbesondere wenn Gleitsichtgläser zur Korrektur eines dann ermittelten Sehfehlers erforderlich sind, eine Anpassung der Gleitsichtgläser an eine Brillenfassung manuell erfolgen muss. So sind auch Geräte bekannt, die eine optische Erfassung von Augen des Probanden zusammen mit einer Brillenfassung mit einer entsprechenden Vermessung ermöglichen. Diese Geräte müssen jedoch unabhängig von dem Sehprüfsystem beschafft und eingesetzt werden, was zusätzliche Kosten und einen hohen Zeitaufwand zur Folge hat.

Aus der EP 2 772 795 A1 ist ein Verfahren zur Bestimmung einer Lage einer Brillenfassung relativ zu den Augen eines Probanden bekannt. Die dazu verwendete Vorrichtung kann bspw. ein Tablet-Computer sein, wobei auf einer Kamera des Tablet-Computers ein Strahlenteiler angeordnet ist, um so eine stereoskopische Aufnahme des Probanden mit der Brillenfassung zu erhalten. Auf einer der Kamera abgewandten Rückseite des Tablet-Computers werden die aufgenommenen Bilder auf einem Anzeigegerät bzw. Monitor dargestellt. Darüber hinaus wird mittels Bildverarbeitung die Brillenfassung erkannt und Referenzpunkte an der Brillenfassung automatisiert ausgewählt. Mittels der Bildverarbeitung kann auch eine Reihe weiterer Parameter, wie bspw. ein Pupillenabstand, deren Lage relativ zur Brillenfassung, ein Abstand der Brillengläser, deren Höhe und auch eine horizontale und vertikale Neigung der Brillengläser in der Brillenfassung sowie ein Abstand von Brillengläsern und Auge bestimmt werden.

Die WO 2004/068216 A1 betrifft ebenfalls ein Verfahren zur Bestimmung einer Position einer Brillenfassung relativ zu den Augen eines Probanden. Auch hier erfolgt die Aufnahme des Probanden mit der Brillenfassung mittels einer Kamera, wobei ergänzend eine Beleuchtung der Augen und der Brillenfassung über einen Strahlenteiler im Strahlengang der Kamera vorgesehen ist. Auch erfolgt eine Darstellung der Erfassungssituation auf einem Monitor, wobei eine Vermessung bzw. Positionsbestimmung mit Hilfe des Monitors durchgeführt werden kann.

Die DE 10 2011 009646 A1 beschreibt eine Vorrichtung in Art eines Tablet-Computers, welche als ein sogenanntes Videozentriersystem ausgebildet ist. Auch hier erfolgt mittels einer Kamera des Videozentriersystems eine Aufnahme eines Probanden mit einer Brillenfassung, wobei ein aufgenommenes Bild auf einem Monitor des Videozentriersystems auf einer der Kamera gegenüberliegenden Rückseite dem Bediener dargestellt wird. Weiter werden mittels Bildverarbeitung eine Reihe von Parametern zur Lage der Brillenfassung relativ zu den Augen des Probanden ermittelt. Darüber hinaus ist vorgesehen, dem Probanden auf einem weiteren, an einer Vorderseite des Videozentriersystems befindlichen Monitor Zeichen oder einen Text wiederzugeben. Die Darstellung dieser Zeichen dient insbesondere zur Steuerung der Blickrichtung des Probanden relativ zu der Kamera.

Der vorliegenden Erfindung liegt daher die Aufgabe zugrunde, ein Verfahren zum Überprüfen der Augen eines Probanden mit einem Sehprüfsystem vorzuschlagen, mittels dem eine Korrektur von Fehlsichtigkeiten einfacher möglich ist.

Diese Aufgabe wird durch ein Verfahren mit den Merkmalen des Anspruchs 1 gelöst. Bei dem erfindungsgemäßen Verfahren zum Überprüfen der Augen eines Probanden mit einem Sehprüfsystem werden zumindest einem Auge des Probanden mittels eines Bildschirms eines Anzeigegeräts des Sehprüfsystems Sehprüfzeichen visualisiert, wobei mit einer Kamera einer Kameravorrichtung des Anzeigegeräts Augen des Probanden zusammen mit einer Brillenfassung des Sehprüfsystems aufgenommen werden, wobei mit einer Steuervorrichtung des Sehprüfsystems das Anzeigegerät gesteuert wird, wobei mit der Steuervorrichtung eine Position der Brillenfassung relativ zu den Augen mittels Bildverarbeitung bestimmt wird, wobei in einem ersten Schritt mittels des Sehprüfsystems eine subjektive Refraktion des Auges des Probanden bestimmt wird und in einem zweiten Schritt mittels des Sehprüfsystems die Position der Brillenfassung relativ zu den Augen bestimmt wird, wobei der zweite Schritt unmittelbar auf den ersten Schritt folgt.

Dadurch, dass einem Auge des Probanden Sehprüfzeichen visualisiert beziehungsweise dargestellt werden, wird ein Sehtest durchgeführt, mittels dem dann subjektive Refraktionswerte des Auges bestimmt werden. Anhand der subjektiven Refraktionswerte des Auges beziehungsweise der Augen des Probanden werden Brillengläser, beispielsweise Gleitsichtgläser, zur jeweils Korrektur einer Fehlsichtigkeit des Auges ausgewählt. Weiter erfolgt eine Anpassung eines Brillengestells bzw. einer Brillenfassung an einen Kopf des Probanden, wobei mit der Kamera die Augen des Probanden zusammen mit der angepassten Brillenfassung aufgenommen werden. Das Sehprüfsystem umfasst daher auch die Brillenfassung. Die Steuervorrichtung beziehungsweise ein Steuergerät des Sehprüfsystems erlaubt eine Steuerung des Anzeigegerätes beziehungsweise des Bildschirms, beispielsweise auch zur Darstellung von Sehprüfzeichen. Die Steuervorrichtung umfasst Mittel zur Datenverarbeitung, die eine Bildverarbeitung der mit der Kamera aufgenommenen Bilddatensätze ermöglichen. Die Aufnahme der Kamera von den Augen des Probanden zusammen mit der Brillenfassung wird nun von der Steuervorrichtung verarbeitet, derart, dass eine Position der Brillenfassung relativ zu den Augen des Probanden bestimmt wird. Insbesondere erfolgt eine Berechnung beziehungsweise Abstandsmessung jeweils der Pupille eines Auges relativ zu einer Innenkontur der Brillenfassung beziehungsweise Brillenglasfassung. Auf Basis dieser Positionsbestimmung kann dann die erforderliche Lage des Brillenglases in der Brilleninnenfassung von der Steuervorrichtung bestimmt werden. Eine gesonderte, manuelle Vermessung ist dann nicht mehr erforderlich. Auch wird kein weiteres, zusätzliches Gerät benötigt, um diese Messung vorzunehmen. Vielmehr kann alleine das Anzeigegerät des Sehprüfsystems zur Bestimmung der Position der Brillenfassung relativ zu den Augen beziehungsweise Pupillen des Probanden bereits im Rahmen eines Sehtests genutzt werden. Insgesamt kann so eine Anpassung einer Brille beziehungsweise Korrektur von Fehlsichtigkeiten kostengünstig und mit geringem Zeitaufwand durchgeführt werden.

Ein subjektiver Refraktionswert der jeweiligen Augen kann mit einer Messbrille oder einem Phoropter ermittelt werden. Auch kann der Phoropter oder die Messbrille Farbfilter oder Polarisationsfilter aufweisen, die jeweils an eine Farbdarstellung und/oder eine Polarisation des Bildschirms angepasst sind, sodass monokulare oder binokulare Sehtests durchgeführt werden können. Wenn beispielsweise ein Phoropter oder eine Messbrille mit linearer oder zirkularer Polarisation vorhanden ist, kann das Anzeigegerät des Sehprüfsystems so ausgewählt werden, dass dieses übereinstimmend mit dem Phoropter oder der Messbrille polarisiert ist. Eine Korrektur einer Polarisation, beispielsweise mit einer λ-Viertel-Folie, ist somit nicht erforderlich.

Erfindungsgemäß wird in einem ersten Schritt mittels des Sehprüfsystems eine subjektive Refraktion des Auges des Probanden bestimmt, und in einem zweiten Schritt wird mittels des Sehprüfsystems die Position der Brillenfassung relativ zu den Augen bestimmt, wobei der zweite Schritt unmittelbar auf den ersten Schritt folgt. Der in dem ersten Schritt durchgeführte Sehtest ist dann unmittelbar auf eine Anpassung des Brillengestells zusammen mit den Brillengläsern erweitert. Der betreffende Proband kann dann beispielsweise zur Anpassung der Brillengläser auf einem Stuhl sitzen bleiben und muss keinen Ortswechsel vornehmen, um an ein anderes Gerät zu gelangen.

Die Bestimmung der Position kann zur Relativpositionierung beziehungsweise Ausrichtung von Gleitsichtgläsern innerhalb der Brillenfassung verwendet werden. Die Gleitsichtgläser können dann über die Brillenfassung besonders genau relativ zu den Augen eines Probanden ausgerichtet werden.

In einer Ausführungsform des Verfahrens kann es vorgesehen sein, dass aus einer Anzahl von Brillenfassungen eine Brillenfassung ausgewählt wird, wobei von der Steuervorrichtung ein Referenzpunkt an der ausgewählten Brillenfassung mittels Bildverarbeitung bestimmt werden kann. Beispielsweise kann ein Proband aus einer Vielzahl von Brillenfassungen eine von ihm individuell bevorzugte Brillenfassung auswählen, zu der dann Brillengläser angefertigt beziehungsweise zur Korrektur von Sehfehlern ausgewählt werden. Zur Relativpositionierung von der Brillenfassung und den Pupillen der Augen kann dann die Steuervorrichtung mittels Bildverarbeitung eine Lage der Pupillen relativ zu der Brillenfassung detektieren. Hier kann es vorteilhaft sein, wenn die Steuervorrichtung an der ausgewählten Brillenfassung einen Referenzpunkt bestimmt, über den die Relativposition von Brillenfassung und Pupillen beschrieben beziehungsweise definiert werden kann. Prinzipiell kann dieser Referenzpunkt an jeder beliebigen Stelle der Brillenfassung angeordnet sein. Auch kann es vorgesehen sein, beispielsweise eine Mehrzahl von Referenzpunkten an der Brillenfassung zu bestimmen. Die Referenzpunkte können relativ zueinander, bezogen auf die Brillenfassung symmetrisch an der Brillenfassung positioniert sein.

Weiter kann die Steuervorrichtung mittels Bildverarbeitung sichtbare konstruktive Details der Brillenfassung bestimmen und diese einem Referenzpunkt zuordnen. Sichtbar konstruktive Details der Brillenfassung können beispielsweise Konturen der Brillenfassung, sichtbare Hell-/Dunkelunterschiede oder Farbunterschiede in einer Aufnahme beziehungsweise einem Bilddatensatz sein. Auch können Details, wie Schrauben, Befestigungspunkte oder dergleichen, ein konstruktives Detail der Brillenfassung sein. Diese sichtbaren konstruktiven Details können mittels Bildverarbeitung erkannt werden, wobei die Steuervorrichtung dann den jeweiligen Details einen Referenzpunkt zuordnen kann. Optional ist es möglich, den konstruktiven Details manuell einen Referenzpunkt zuzuordnen.

Die Steuervorrichtung kann auch eine Datenbank mit Datensätzen von Brillenfassungen aufweisen, wobei die Steuervorrichtung weiter einen mit der Kamera aufgenommenen Bilddatensatz der Brillenfassung am Kopf des Probanden mit den Bilddatensätzen der Datenbank vergleichen kann, wobei die Steuervorrichtung bei übereinstimmenden Bilddatensätzen der Brillenfassung am Kopf des Probanden einen Referenzpunkt zuordnen kann. Durch einen Bildvergleich der Aufnahme der Brillenfassung am Kopf des Probanden mit Aufnahmen von Brillenfassungen in der Datenbank kann die Brillenfassung am Kopf des Probanden gegebenenfalls in der Form erkannt werden, dass dieser Brillenfassung ein Hersteller, Modell und/oder Typ zugeordnet werden kann. Weiter kann vorgesehen sein, dass die Bilddatensätze in der Datenbank beziehungsweise die Brillenfassungen jeweils bereits Referenzpunkte aufweisen. So ist es möglich, eine beliebige Brillenfassung mit der Kamera aufzunehmen, dieser dann einen Referenzpunkt zuzuweisen und den entsprechenden Bilddatensatz in der Datenbank zu speichern. Diese Brillenfassung ist dann folglich für eine Verwendung mit dem Sehprüfsystem kalibriert. Zusammen mit dem Bilddatensatz können noch weitere Datensätze mit beispielsweise Brillenfassungsmaßen, Herstellerangaben usw. in der Datenbank gespeichert werden.

Die Brillenfassung beziehungsweise die vom Probanden ausgewählte Brillenfassung kann mit einer Referenzmarkierung versehen werden, wobei die Steuervorrichtung die Referenzmarkierung als einen Referenzpunkt bestimmen kann. Die Referenzmarkierung kann beispielsweise in Form eines Aufklebers oder einer anderen Art von visueller Markierung an der Brillenfassung temporär angebracht werden. Die Steuervorrichtung kann dann die Referenzmarkierungen erkennen und als Referenzpunkt festlegen. Auch bei unterschiedlich ausgebildeten Brillenfassungen kann die Referenzmarkierung stets an gleichen Positionen, wie beispielsweise einem Scheitelpunkt der Brillenfassung, angebracht werden. Die Referenzmarkierung kann aber auch eine horizontale und/oder vertikale Skala sein, die an der Brillenfassung angebracht wird.

Ebenso kann die Brillenfassung von einer Messbrille ausgebildet werden. Die Abmessungen der Messbrille können dann bereits in der Steuervorrichtung gespeichert sein. So wird es auch möglich, während eines Sehtests bereits eine Position der Brillenfassung beziehungsweise Messbrille relativ zu den Augen des Probanden zu bestimmen und diese Positionsdaten später zur Anpassung von Brillengläsern zu nutzen. Auch hier ist es möglich, konstruktive Details der Messbrille als eine Referenzmarkierung zur Festlegung eines Referenzpunktes zu verwenden.

Relativ bezogen auf einen Kopf des Probanden kann eine frontale Bildaufnahme erfolgen und vorzugsweise kann nachfolgend eine sagittale Bildaufnahme erfolgen oder umgekehrt. Die frontale Bildaufnahme dient dann zur Positionsbestimmung der Brillenfassung relativ zu den Pupillen des Probanden, wobei die sagittale Bildaufnahme zur beispielsweise Bestimmung eines Hornhaut-Scheitelabstandes oder einer Brillenfassungsvorneigung dienen kann. Die sagittale Bildaufnahme kann einfach dadurch erfolgen, dass der Proband seinen Kopf um 90° relativ zu einer optischen Achse der Kamera verdreht beziehungsweise quer neigt. Auch können zwei sagittale Bildaufnahmen von jeweils beiden Seiten des Kopfes mittels der Kamera erfasst und von der Steuervorrichtung weiterverarbeitet werden. Im Falle einer sagittalen Bildaufnahme kann wie bei einer frontalen Bildaufnahme die Steuervorrichtung Referenzpunkte bestimmen.

Vorteilhaft ist es, wenn ein Relativabstand von Brillenfassung und Kamera bestimmt wird. Die Bestimmung des Relativabstandes kann beispielsweise mittels Triangulation erfolgen. So kann zur Berechnung des Relativabstandes mittels der Steuervorrichtung eine Skala an der Brillenfassung oder ein bekannter Referenzpunktabstand an der Brillenfassung genutzt werden. Weiter kann auch ein Relativabstand, beispielsweise bei einer ortsfesten Positionierung des Anzeigegeräts beziehungsweise der Kamera und des Probanden bei einem Sehtest als bekannt vorausgesetzt werden, sodass der Relativabstand dann in der Steuervorrichtung gespeichert werden kann. Der Relativabstand kann dann wiederum zur Berechnung von beispielsweise eines Pupillenabstandes oder von Referenzpunkt und Pupille verwendet werden.

Im Rahmen des Verfahrens können auch Brillenfassungsmaße, eine Pupillendistanz, Zentrierpunktabstände, eine Anpasshöhe, ein Hornhaut-Scheitelabstand, eine Fassungsvorneigung und/oder ein Fassungsscheibenwinkel mit der Kamera erfasst und mit der Steuervorrichtung mittels Bildverarbeitung ermittelt werden.

Auch kann eine vergleichende Positionsbestimmung mit einer Referenzbrillenfassung des Sehprüfsystems und einer Originalbrille des Probanden durchgeführt werden. Die Originalbrille des Probanden kann eine Brille sein, die bereits an Augen des Probanden angepasst ist. Die vergleichende Positionsbestimmung beziehungsweise Messung ermöglicht dann eine Überprüfung einer Eignung der Originalbrille oder der Referenzbri llenfass ung.

Besonders vorteilhaft ist es auch, wenn die Brillenfassung mit Referenzmarkierungen verwendet wird, und vorzugsweise horizontale und vertikale Skalen, aufweist. Beispielsweise kann die Brillenfassung eine Messbrille sein, die diese Skalen bereits aufweist. Eine Lage einer Pupille relativ zu der Brillenfassung beziehungsweise Skala ist dann besonders einfach bestimmbar.

Der Bildschirm und die Kamera können in einem gemeinsamen Gehäuse des Anzeigegeräts angeordnet werden. Das Sehprüfsystem wird dann auch besonders einfach handhabbar, da nicht mehrere miteinander gekoppelte und dennoch voneinander beabstandete Geräte zur Durchführung eines Sehtests und zur Anpassung einer Brille benötigt werden.

Der Bildschirm kann hintergrundbeleuchtet werden, wobei das Anzeigegerät als ein ortsfestes Ferntest-Anzeigegerät verwendet werden kann, dessen Anzeigeflächengröße für Sehtests mit einem Sehabstand von 3 m bis 10 m, vorzugsweise von 4 m bis 8 m ausgebildet ist, und/oder als ein mobil handhabbares Nahtest-Anzeigegerät verwendet werden kann, dessen Anzeigeflächengröße für Sehtests mit einem Sehabstand von 10 cm bis 3 m, vorzugsweise von 30 cm bis 1 m ausgebildet ist. Das Ferntest-Anzeigegerät kann dann zur Darstellung von Sehprüfzeichen zum Testen des Fernsehens und das Nahtest-Anzeigegerät zur Darstellung von Sehprüfzeichen zum Testen des Nahsehens genutzt werden. Beide Anzeigegeräte können für sich alleine oder auch in Kombination miteinander als Sehprüfsystem verwendet werden. Das Ferntest-Anzeigegerät kann vorzugsweise in dem oben angegebenen Sehabstand relativ zu dem Probanden ortsfest aufgestellt oder an einer Wand montiert sein. Wenn der Proband relativ zum Ferntest-Anzeigegerät in einer definierten Sehentfernung zur Durchführung von Sehtests platziert wird, kann die Sehentfernung zu dem Ferntest-Anzeigegerät genau bestimmt werden. Auch kann dann eine Anzeigeflächengröße des Ferntest-Anzeigegerätes um ein Vielfaches größer sein als eine Anzeigeflächengröße des Nahtest-Anzeigegeräts, da gegebenenfalls auf der Anzeigefläche des Nahtest-Anzeigegeräts vergleichsweise größere Sehprüfzeichen dargestellt werden. Das Nahtest-Anzeigegerät kann auch mobil handhabbar sein, sodass dieses in einem nahezu beliebigen Abstand innerhalb des oben angegebenen Abstands relativ zu den Augen des Probanden von einer Bedienperson oder dem Proband gehalten oder platziert werden kann. Sowohl das Ferntest-Anzeigegerät als auch das Nahtest-Anzeigegerät können über die Steuervorrichtung von einer Bedienperson ferngesteuert werden. Die Steuervorrichtung kann dann ein Steuergerät zur Fernsteuerung aufweisen.

Nachfolgend wird eine bevorzugte Ausführungsform der Erfindung unter Bezugnahme auf die beigefügten Zeichnungen näher erläutert.

Es zeigen:
- **Fig. 1:**: eine Vorderansicht eines Anzeigegeräts;
- **Fig. 2:**: eine Vorderansicht einer Brillenfassung;
- **Fig. 3:**: eine Seitenansicht der Brillenfassung.

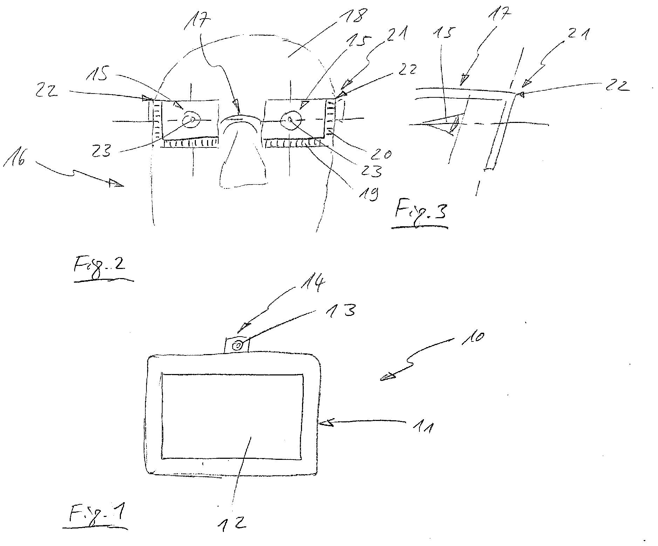

Eine Zusammenschau der **Fig. 1** bis **3** zeigt ein Sehprüfsystem 10 mit einem Anzeigegerät 11, welches einen hintergrundbeleuchteten Bildschirm 12, in Art eines Fernsehgeräts oder eines Tablet-Computers aufweist. Das Anzeigegerät 11 verfügt über eine Kameravorrichtung 14 mit einer Kamera 13, welche Augen 15 eines Probanden 16 zusammen mit einer Brillenfassung 17 beziehungsweise Brille aufnehmen kann. Die Brillenfassung 17 wird vom Sehprüfsystem 10 umfasst beziehungsweise ist Bestandteil desselben. Mit dem Bildschirm 12 werden dem Probanden 16 zunächst Sehprüfzeichen dargestellt, um einen subjektiven Refraktionswert der jeweiligen Augen 15 mit der Brillenfassung 17, die auch eine Messbrille sein kann, oder einem hier nicht dargestellten Phoropter zu ermitteln. Unmittelbar nachfolgend wird mit dem Anzeigegerät 11 und der Brillenfassung 17 eine Anpassung einer Gleitsichtbrille beziehungsweise einer Ausrichtung von Gleitsichtgläsern relativ zu den Augen 15 vorgenommen. Diese erfolgt durch eine frontale Aufnahme der Augen 15 des Probanden 16 zusammen mit der Brillenfassung 17. Optional kann der Proband 16 seinen Kopf 18 um 90° drehen, sodass entsprechend der Darstellung in **Fig. 3** eine seitliche Aufnahme des um 90° gedrehten Kopfes 18 zusammen mit der Brillenfassung 17 mittels der Kamera 13 erfolgen kann.

Die Brillenfassung 17 weist horizontale Skalen 19 und vertikale Skalen 20 auf. Anhand der Skalen 19 und 20 kann mittels Bildverarbeitung ein Abstand von der Kamera 13 zu der Brillenfassung 17 durch Triangulation errechnet werden. Weiter kann auch eine Ecke 21 an der Brillenfassung 17 einen Referenzpunkt 22 ausbilden, wobei ein bereits bekannter Relativabstand der Referenzpunkte 22 zur Berechnung des Abstands zu der Kamera 13 herangezogen werden kann. Weiter kann anhand der Skalen 19 und 20 ein Sitz der Brillenfassung 17 an dem Kopf 18 des Probanden 16 überprüft werden. Pupillen 23 der Augen 15 können ebenfalls mittels Bildverarbeitung erkannt werden, sodass eine Position der Brillenfassung 17 relativ zu den Pupillen 23 beziehungsweise den Augen 15 leicht bestimmt werden kann. Prinzipiell kann es sich neben der hier dargestellten Brillenfassung 17 um jede beliebige Brillenfassung beziehungsweise Brille handeln, mit welcher eine Positionsbestimmung möglich ist. Insbesondere können mit der Kamera 13 Fassungsmaße, eine Pupillendistanz, Zentrierpunktabstände, eine Anpasshöhe, ein Hornhaut-Scheitelabstand, eine Fassungsvorneigung und ein Fassungsscheibenwinkel erfasst und mit der hier nicht dargestellten Steuervorrichtung mittels Bildverarbeitung ermittelt werden.

## Patentansprüche

1. Verfahren zum Überprüfen der Augen eines Probanden mit einem Sehprüfsystem (10), wobei zumindest einem Auge (15) des Probanden (16) mittels eines Bildschirms (12) eines Anzeigegeräts (11) des Sehprüfsystems Sehprüfzeichen visualisiert werden, wobei mit einer Kamera (13) einer Kameravorrichtung (14) des Anzeigegeräts Augen des Probanden zusammen mit einer Brillenfassung (17) des Sehprüfsystems aufgenommen werden, wobei mit einer Steuervorrichtung des Sehprüfsystems das Anzeigegerät gesteuert wird, wobei mit der Steuervorrichtung eine Position der Brillenfassung relativ zu den Augen mittels Bildverarbeitung bestimmt wird,
**dadurch gekennzeichnet,**
**dass** in einem ersten Schritt mittels des Sehprüfsystems eine subjektive Refraktion des Auges des Probanden durch das Visualisieren der Sehprüfzeichen bestimmt wird und in einem zweiten Schritt mittels des Sehprüfsystems die Position der Brillenfassung relativ zu den Augen bestimmt wird, wobei der zweite Schritt unmittelbar auf den ersten Schritt folgt.

2. Verfahren nach Anspruch 1,
**dadurch gekennzeichnet,**
**dass** ein subjektiver Refraktionswert der jeweiligen Augen (15) mit einer Messbrille oder einem Phoropter ermittelt wird.

3. Verfahren nach Anspruch 1 oder 2,
**dadurch gekennzeichnet,**
**dass** die Bestimmung der Position zur Relativpositionierung von Gleitsichtgläsern innerhalb der Brillenfassung (17) verwendet wird.

4. Verfahren nach einem der vorangehenden Ansprüche,
**dadurch gekennzeichnet,**
**dass** aus einer Anzahl von Brillenfassungen eine Brillenfassung (17) ausgewählt wird, wobei von der Steuervorrichtung ein Referenzpunkt (22) an der ausgewählten Brillenfassung mittels Bildverarbeitung bestimmt wird.

5. Verfahren nach einem der vorangehenden Ansprüche,
**dadurch gekennzeichnet,**
**dass** die Steuervorrichtung mittels Bildverarbeitung sichtbare konstruktive Details (21) der Brillenfassung (17) bestimmt und diesen einen Referenzpunkt (22) zuordnet.

6. Verfahren nach einem der vorangehenden Ansprüche,
**dadurch gekennzeichnet,**
**dass** die Steuervorrichtung eine Datenbank mit Bilddatensätzen von Brillenfassungen aufweist, wobei die Steuervorrichtung einen mit der Kamera (13) aufgenommenen Bilddatensatz der Brillenfassung (17) mit den Bilddatensätzen der Datenbank vergleicht, wobei die Steuervorrichtung bei übereinstimmenden Bilddatensätzen der Brillenfassung einen Referenzpunkt (22) zuordnet.

7. Verfahren nach einem der vorangehenden Ansprüche,
**dadurch gekennzeichnet,**
**dass** die Brillenfassung (17) mit einer Referenzmarkierung (19, 20) versehen wird, wobei die Steuervorrichtung die Referenzmarkierung als einen Referenzpunkt (22) bestimmt.

8. Verfahren nach einem der vorangehenden Ansprüche,
**dadurch gekennzeichnet,**
**dass** die Brillenfassung (17) von einer Messbrille ausgebildet wird.

9. Verfahren nach einem der vorangehenden Ansprüche,
**dadurch gekennzeichnet,**
**dass** relativ bezogen auf einen Kopf (18) des Probanden (16) eine frontale Bildaufnahme erfolgt und vorzugsweise nachfolgend eine sagittale Bildaufnahme erfolgt.

10. Verfahren nach einem der vorangehenden Ansprüche,
**dadurch gekennzeichnet,**
**dass** ein Relativabstand von Brillenfassung (17) und Kamera (13) bestimmt wird.

11. Verfahren nach einem der vorangehenden Ansprüche,
**dadurch gekennzeichnet,**
**dass** Brillenfassungsmaße, eine Pupillendistanz, Zentrierpunktabstände, eine Anpasshöhe, ein Hornhaut-Scheitelabstand, eine Fassungsvorneigung und/oder ein Fassungsscheibenwinkel mit der Kamera (13) erfasst und mit dem Steuervorrichtung mittels Bildverarbeitung ermittelt werden.

12. Verfahren nach einem der vorangehenden Ansprüche,
**dadurch gekennzeichnet,**
**dass** eine vergleichende Positionsbestimmung mit einer Referenzbrillenfassung (17) des Sehprüfsystems (10) und einer Originalbrille des Probanden durchgeführt wird.

13. Verfahren nach einem der vorangehenden Ansprüche,
**dadurch gekennzeichnet,**
**dass** die Brillenfassung (17) mit Referenzmarkierungen verwendet wird, und vorzugsweise horizontale und vertikale Skalen (19, 20), aufweist.

14. Verfahren nach einem der vorangehenden Ansprüche,
**dadurch gekennzeichnet,**
**dass** der Bildschirm (12) und die Kamera (13) in einem gemeinsamen Gehäuse des Anzeigegeräts (11) angeordnet werden.

15. Verfahren nach einem der vorangehenden Ansprüche,
**dadurch gekennzeichnet,**
**dass** der Bildschirm (12) hintergrundbeleuchtet wird, wobei das Anzeigegerät (11) als ein ortsfestes Ferntest-Anzeigegerät verwendet wird, dessen Anzeigeflächengröße für Sehtests mit einem Sehabstand von 3 m bis 10 m ausgebildet ist und/oder als ein mobil handhabbares Nahtest-Anzeigegerät verwendet wird, dessen Anzeigeflächengröße für Sehtests mit einem Sehabstand von 10 cm bis 3 m ausgebildet ist.

## Claims

1. A method for examining a subject's eyes with an eye examining system (10), eye examination symbols being made visible to at least one of the subject's (16) eyes (15) by means of a monitor (12) of a display device (11) of the eye examining system, the subject's eyes being recorded in conjunction with a spectacle frame (17) of the eye examining system using a camera (13) of a camera device (14) of the display device, the display device being controlled by a control apparatus of the eye examining system, a position of the spectacle frame being determined relative to the eyes by means of image processing using the control apparatus,
**characterized in that**
in a first step, a subjective refraction of the subject's eye is determined by means of the eye examining system by making visible the eye examination symbols and, in a second step, the position of the spectacle frame is determined relative to the eyes by means of the eye examining system, the first step being directly followed by the second step.

2. The method according to claim 1,
**characterized in that**
a subjective refraction of the corresponding eyes (15) is determined using a trial frame or a phoropter.

3. The method according to claim 1 or 2,
**characterized in that**
the determined position is used for relatively positioning varifocal lenses within the spectacle frame (17).

4. The method according to any one of the preceding claims,
**characterized in that**
a spectacle frame (17) is chosen from a plurality of spectacle frames, a reference point (22) being determined at the chosen spectacle frame by the control apparatus by means of image processing.

5. The method according to any one of the preceding claims,
**characterized in that**
the control apparatus determines visible constructive details (21) of the spectacle frame (17) and allocates a reference point (22) thereto by means of image processing.

6. The method according to any one of the preceding claims,
**characterized in that**
the control apparatus comprises a data bank having image data sets of spectacle frames, the control apparatus comparing an image data set of the spectacle frame (17) recorded by the camera (13) to the image data sets of the data bank, the control apparatus allocating a reference point (22) when image data sets of the spectacle frame correspond to each other.

7. The method according to any one of the preceding claims,
**characterized in that**
the spectacle frame (17) is provided with a reference marking (19, 20), the control apparatus determining the reference marking as a reference point (22).

8. The method according to any one of the preceding claims,
**characterized in that**
the spectacle frame (17) is formed by a trial frame.

9. The method according to any one of the preceding claims,
**characterized in that**
a frontal image is recorded relatively relating to the subject's (16) head (18) and preferably a sagittal image is recorded thereafter.

10. The method according to any one of the preceding claims,
**characterized in that**
a relative distance is determined between the spectacle frame (17) and the camera (13).

11. The method according to any one of the preceding claims,
**characterized in that**
spectacle frame measurements, a pupil distance, centering point distances, an adjusting height, a corneal vertex distance, a frame tilt, and/or a frame lens tilt are recorded by the camera (13) and are determined by the control apparatus by means of image processing.

12. The method according to any one of the preceding claims,
**characterized in that**
a position is comparatively determined using a reference spectacle frame (17) of the eye examining system (10) and a subject's own pair of spectacles.

13. The method according to any one of the preceding claims,
**characterized in that**
the spectacle frame (17) comprises reference markings, preferably horizontal and vertical scales (19, 20).

14. The method according to any one of the preceding claims,
**characterized in that**
the monitor (12) and the camera (13) are arranged in a shared housing of the display device (11).

15. The method according to any one of the preceding claims,
**characterized in that**
the monitor (12) is backlit, the display device (11) being a stationary display device for testing farsightedness, whose display surface size is designed for conducting eye examinations at a seeing distance of 3 m to 10 m, and/or is a display device for testing nearsightedness for mobile use, whose display surface size is designed for conducting eye examinations at a seeing distance of 10 cm to 3 m.

## Revendications

1. Procédé d'examen des yeux d'un sujet à l'aide d'un système (10) d'examen des yeux, des symboles d'examen des yeux étant rendus visibles à au moins un des yeux (15) du sujet (16) au moyen d'un écran (12) d'un dispositif d'affichage (11) du système d'examen des yeux, les yeux du sujet étant enregistrés en conjonction avec une monture de lunettes (17) du système d'examen des yeux à l'aide d'une caméra (13) d'un dispositif de caméra (14) du dispositif d'affichage, le dispositif d'affichage étant commandé par un appareil de commande du système d'examen des yeux, une position de la monture de lunettes étant déterminée par rapport aux yeux au moyen d'un traitement d'image utilisant l'appareil de commande, **caractérisé en ce que**,
dans une première étape, une réfraction subjective de l'oeil du sujet est déterminée au moyen du système d'examen des yeux en rendant visibles les symboles d'examen des yeux et **en ce que**, dans une deuxième étape, la position de la monture de lunettes est déterminée par rapport aux yeux au moyen du système d'examen des yeux, la première étape étant directement suivie de la deuxième étape.

2. Procédé selon la revendication 1,
**caractérisée en ce**
**qu'**une réfraction subjective des yeux (15) correspondants est déterminée à l'aide d'une monture d'essai ou d'un phoroptère.

3. Procédé selon la revendication 1 ou la revendication 2,
**caractérisé par le fait que**
la position déterminée est utilisée pour le positionnement relatif des verres progressifs dans la monture de lunettes (17).

4. Procédé selon l'une quelconque des revendications précédentes, **caractérisé en ce**
**qu'**une monture de lunettes (17) est choisie parmi une pluralité de montures de lunettes, un point de référence (22) étant déterminé sur la monture de lunettes choisie par l'appareil de commande au moyen d'un traitement d'images.

5. Procédé selon l'une quelconque des revendications précédentes, **caractérisé en ce que**
l'appareil de commande détermine des détails (21) constructifs visibles de la monture de lunettes (17) et leur attribue un point de référence (22) au moyen d'un traitement d'images.

6. Procédé selon l'une quelconque des revendications précédentes, **caractérisé en ce que**
l'appareil de commande comprend une banque de données contenant des ensembles de données d'images de montures de lunettes, l'appareil de commande comparant un ensemble de données d'images de la monture de lunettes (17) enregistré par la caméra (13) aux ensembles de données d'images de la banque de données, l'appareil de commande attribuant un point de référence (22) lorsque les ensembles de données d'images de la monture de lunettes correspondent l'un à l'autre.

7. Procédé selon l'une quelconque des revendications précédentes, **caractérisé en ce que**
la monture de lunettes (17) est pourvue d'un marquage de référence (19, 20), l'appareil de commande déterminant le marquage de référence comme point de référence (22).

8. Procédé selon l'une quelconque des revendications précédentes, **caractérisé en ce que**
la monture de lunettes (17) est constituée d'une monture d'essai.

9. Procédé selon l'une quelconque des revendications précédentes, **caractérisé en ce**
**qu'**une image frontale est enregistrée relativement à la tête (18) du sujet (16) et, de préférence, une image sagittale est enregistrée par la suite.

10. Procédé selon l'une quelconque des revendications précédentes, **caractérisé en ce**
**qu'**une distance relative est déterminée entre la monture de lunettes (17) et la caméra (13).

11. Procédé selon l'une quelconque des revendications précédentes, **caractérisé en ce que**
des mesures de la monture de lunettes, une distance pupillaire, des distances entre les points de centrage, une hauteur de réglage, une distance entre le sommet de la cornée, une inclinaison de la monture et/ou une inclinaison de la lentille de la monture sont enregistrées par la caméra (13) et sont déterminées par l'appareil de commande au moyen d'un traitement d'image.

12. Procédé selon l'une quelconque des revendications précédentes, **caractérisée en ce**
**qu'**une position est déterminée comparativement à l'aide d'une monture de lunettes de référence (17) du système d'examen de l'oeil (10) et de la propre paire de lunettes du sujet.

13. Procédé selon l'une quelconque des revendications précédentes, **caractérisé en ce que**
la monture de lunettes (17) comporte des marquages de références, de préférence des échelles horizontales et verticales (19, 20).

14. Procédé selon l'une des revendications précédentes,
**caractérisée en ce que**
l'écran (12) et la caméra (13) sont disposés dans un boîtier commun du dispositif d'affichage (11).

15. Procédé selon l'une quelconque des revendications précédentes, **caractérisé en ce que**
l'écran (12) est rétroéclairé, le dispositif d'affichage (11) étant un dispositif d'affichage fixe pour tester l'hypermétropie, dont la taille de la surface d'affichage est conçue pour effectuer des examens de vue à une distance de vision de 3 m à 10 m, et/ou est un dispositif d'affichage pour tester la myopie pour une utilisation mobile, dont la taille de la surface d'affichage est conçue pour effectuer des examens de vue à une distance de vision de 10 cm à 3 m.
